(19) Europäisches Patentamt
European Patent Office
Office européen des brevets

(11) **EP 2 620 760 A2**

(12) **EUROPEAN PATENT APPLICATION**

(43) Date of publication:
**31.07.2013 Bulletin 2013/31**

(51) Int Cl.:
*G01N 21/27* (2006.01)   *B01L 3/00* (2006.01)
*G01N 21/03* (2006.01)

(21) Application number: **13152514.9**

(22) Date of filing: **24.01.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **26.01.2012 KR 20120007650**

(71) Applicant: **Samsung Electronics Co., Ltd
Gyeonggi-do 443-742 (KR)**

(72) Inventors:
• **YEO, Yeong Bae**
  **Seoul (KR)**
• **GWON, Se Do**
  **Gyeonggi-do (KR)**
• **CHO, Hee Sung**
  **Gyeonggi-do (KR)**

(74) Representative: **Grünecker, Kinkeldey,
Stockmair & Schwanhäusser
Leopoldstrasse 4
80802 München (DE)**

(54) **Microfluidic device and control method thereof**

(57)    A microfluidic device and control method thereof are provided. The control method of the microfluidic device includes detecting a background signal from a chamber in which a reaction product formed by combining an analyte and a marking material is not accommodated, detecting a detection signal from a chamber in which the reaction product is accommodated, and compensating for the detection signal using the background signal.

FIG. 3

EP 2 620 760 A2

**Description**

BACKGROUND

**1. Field**

Methods and apparatuses consistent with exemplary embodiments relate to a centrifugal force-based microfluidic device which accurately detects a signal discharged from a chamber and a control method thereof.

**2. Description of the Related Art**

A microfluidic device is used to execute a biological or chemical reaction by manipulating a fluid of a small amount.

In general, a microfluidic structure executing one or more independent functions in the microfluidic device may include one or more of each of a chamber accommodating a fluid, a channel in which the fluid flows and a valve to adjust the flow of the fluid. A lab-on-a-chip refers to a device in which such microfluidic structures are arranged on a chip-type substrate, and upon which various steps of processing and operations are executed so as to implement tests including immunoassay reactions or biochemical reactions on a small chip.

In order to transfer the fluid within the microfluidic structure, pressure is required. Examples of such pressure include but are not limited to, capillary pressure or pressure generated by a separate pump. Recently, a disc-type microfluidic device within which microfluidic structures are arranged on a disc-shaped platform to perform a series of processes by moving a fluid using centrifugal force has been proposed. Such a device is referred to a lab CD or a lab-on-a disc.

When used in a method of detecting an analyte, a microfluidic device provides the ability to measure the concentration of a marker combined with the analyte. However, in measuring the concentration of the marker, interference caused by a signal discharged from a detection chamber within the microfluidic device itself or interference caused by a signal discharged from a marker accommodated in another detection chamber of the microfluidic device may influence an inspection result.

SUMMARY

Exemplary embodiments provide a microfluidic device which measures a background signal before a reaction product is accommodated in a chamber, and compensates for a signal measured after the reaction product is accommodated in the chamber using the background signal to improve reliability of inspection, and a control method thereof.

In accordance with an aspect of an exemplary embodiment, there is provided a control method of a microfluidic device, the control method including detecting a background signal from a first chamber disposed within the microfluidic device, wherein the first chamber does not contain a reaction product, the reaction product being a complex of an analyte and a marking material, detecting a detection signal from a second chamber disposed within the microfluidic device, wherein the second chamber contains the reaction product, and compensating for the detection signal by removing the background signal from the detection signal.

The first chamber and the second chamber may be the same chamber.

The first chamber and the second chamber may be different chambers.

The control method may further include transferring the reaction product to the second chamber, after the detection of the background signal from the first chamber. The first and second chambers may be reaction chambers or detection chambers. The marking material may be one selected from the group consisting of a fluorescent material, a metal colloid, an enzyme, latex beads, a noctilucent material and a radioactive isotope.

The compensation of the detection signal may include removing the background signal from the detection signal.

The control method may further include vibrating the microfluidic device to uniformly distribute the marking material transferred to the second chamber, before the detection of the detection signal.

The detection signal may be detected from the second chamber after a designated time from transfer of the reaction product to the second chamber has elapsed.

The designated time may correspond to a time taken to stabilize the flow of the marking material transferred to the second chamber.

The control method may further include detecting the detection signal from the second chamber a plurality of times and calculating the mean value of the detected detection signals, and the detection signal to be compensated for may be the mean value of the plurality of detection signals.

The detection signal may be detected from at least two different detection positions.

In accordance with an aspect of another exemplary embodiment, there is provided a microfluidic device including a first chamber disposed within a platform and in which a reaction product is not accommodated, wherein the reaction product is a complex of an analyte and a marking material, a second chamber disposed within the platform and within which the reaction product is accommodated, a signal detection unit positioned in proximity to the platform for detecting signals emitted from the first chamber and the second chamber, and a controller electrically connected to the signal detection unit that compensates for the signal detected from the second chamber using the signal detected from the first chamber.

In accordance with an aspect of another exemplary embodiment, there is provided a microfluidic device including a first chamber disposed within a platform and within which a reaction product is generated, wherein the reaction product is a complex of an analyte and a marking material, a second chamber disposed within the platform and within which the reaction product transferred from the first chamber is accommodated, a signal detection unit positioned in proximity to the platform for detecting signals emitted from the second chamber, and a controller electrically connected to the signal detection unit that compensates for a signal detected by the signal detection unit after the reaction product is transferred to the second chamber, using a signal detected by the signal detection unit before the reaction product is transferred to the second chamber.

The first chamber and the second chamber may be reaction chambers.

The first chamber may be a reaction chamber and the second chamber may be a detection chamber.

The marking material may be one selected from the group consisting of a fluorescent material, a metal colloid, an enzyme, latex beads, a noctilucent material and a radioactive isotope.

The controller may remove the signal detected from the first chamber from the signal detected from the second chamber.

The controller may remove the signal detected by the signal detection unit before the reaction product is transferred to the second chamber, from the signal detected by the signal detection unit after the reaction product is transferred to the second chamber.

The controller may vibrate the microfluidic device to uniformly disperse the marking material transferred to the second chamber, before the signal detection unit detects the signal after the reaction product is accommodated in the second chamber.

The controller may control the signal detection unit so as to detect the signal from the second chamber after a designated time from transfer of the reaction product to the second chamber has elapsed.

The designated time may correspond to a time taken to stabilize the flow of the marking material transferred to the second chamber.

The controller may control the signal detection unit so as to detect the signal from the second chamber in which the reaction product is accommodated a plurality of times, and compensate for the mean value of the detected detection signals.

The controller may control the signal detection unit so as to detect the signal from at least two different detection positions.

In accordance with an aspect of another exemplary embodiment, there is provided a microfluidic device including a reaction chamber disposed within a platform and in which a reaction product is generated, wherein the reaction product is a complex of an analyte and a marking material, a plurality of detection chambers disposed within the platform and into which the reaction product is transferred through channels connected to the reaction chamber, a signal detection unit positioned in proximity to the platform for detecting a marking signal emitted from the plurality of detection chambers, and an opaque cartridge surrounding each of the plurality of detection chambers.

The cartridge may be formed of a material which does not transmit light.

The cartridge may be formed in a color which does not transmit light.

BRIEF DESCRIPTION OF THE DRAWINGS

The above and/or other aspects will become apparent and more readily appreciated from the following description of exemplary embodiments, taken in conjunction with the accompanying drawings of which:

FIG. 1 is a perspective view schematically illustrating the structure of a microfluidic device in accordance with an exemplary embodiment;
FIG. 2 is a plan view illustrating a microfluidic structure including one analysis unit in the microfluidic device in accordance with the exemplary embodiment;
FIG. 3 is a control block diagram of the microfluidic device in accordance with the exemplary embodiment;
FIGS. 4A to 4C are views illustrating measurement of fluorescent signals at different detection positions several times;
FIGS. 5 and 6 are views illustrating a detection chamber surrounded with a cartridge in accordance with an exemplary embodiment;
FIG. 7 is a cross-sectional view illustrating the cartridge in accordance with another exemplary embodiment.
FIG. 8 is a flowchart illustrating a control method of a microfluidic device in accordance with an exemplary embodiment;
FIG. 9 is a flowchart illustrating a control method of a microfluidic device in accordance with another exemplary embodiment;
FIG. 10 is a flowchart illustrating a control method of a microfluidic device in accordance with another exemplary embodiment; and
FIG. 11 is a flowchart illustrating a control method of a microfluidic device in accordance with yet another exemplary embodiment.

DETAILED DESCRIPTION

Exemplary embodiments will now be described in detail with reference to the accompanying drawings, wherein like reference numerals refer to like elements throughout.

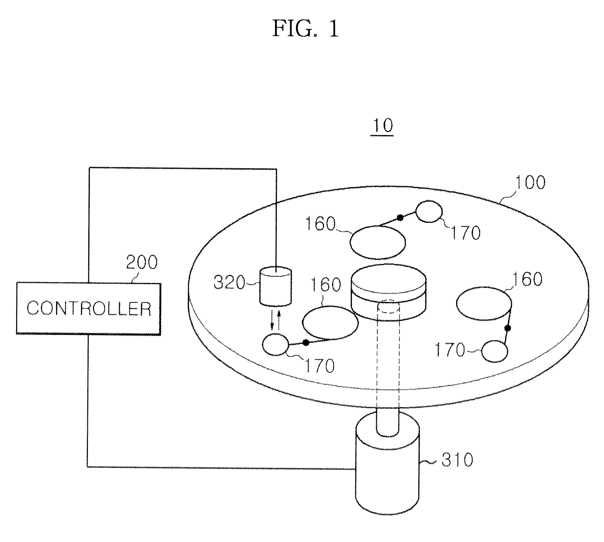

FIG. 1 is a perspective view schematically illustrating the structure of a microfluidic device in accordance with an exemplary embodiment.

With reference to FIG. 1, a microfluidic device 10 in accordance with this exemplary embodiment includes a platform 100 within which microfluidic structures are formed, a drive unit 310 driving the platform 100, reaction chambers 160 and detection chambers 170 formed within the platform 100, a signal detection unit 320 detecting a signal discharged from the detection chamber 170, and a controller 200 controlling the operation of the microfluidic device 10.

The platform 100 may be formed of plastic, such as acryl (PMMA), PDMS, PC, etc., the surface of which is biologically inactive. However, the material of the platform 100 is not limited thereto, and may be formed from any material having properties such as chemical and biological stability, optical transparency, and mechanical processability.

Although the exemplary embodiment shown in FIG. 1 uses a disc-shaped platform 100, the shape of the platform 100 is not limited thereto, and may have any of various shapes, such as a fan shape, which may be seated on a rotatable frame and be rotated.

The drive unit 310 may be a motor, and may provide rotary force to the platform 100, thereby moving fluids accommodated in various chambers provided within the platform 100.

Although one inspection unit that includes one or more microfluidic structures may be provided on a single platform 100, a single platform 100 may be divided into a plurality of regions with microfluidic structures being operated independently of each other within the respective regions in order to improve promptness of inspection and cost efficiency. Each of the plurality of microfluidic structures may be configured to execute different tests so as to simultaneously execute several inspections, or each of the plurality of microfluidic structures may be configures to execute the same inspection.

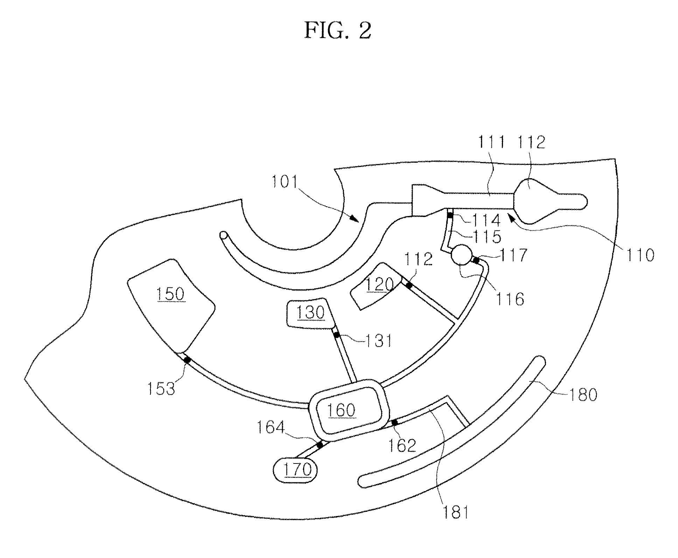

FIG. 2 is a plan view illustrating a microfluidic structure, which includes one analysis unit in the microfluidic device. In this exemplary embodiment, centrifugal force caused by rotation is used as the driving force for moving a fluid therein.

With reference to FIG. 2, one analysis unit provided within the platform 100 includes a sample chamber 101, a reaction chamber 160, a detection chamber 170, a waste chamber 180, and channels connecting the chambers.

The sample chamber 101 provides a space to accommodate a liquid sample, for example, blood. Although not shown in the drawings, the sample chamber 101 may include an inlet through which the sample is injected into the sample chamber 101, an accommodation part to accommodate the sample, and an outlet connected to a sample separation unit 110. The outlet may be configured so as to form capillary pressure to prevent movement of the sample to the sample separation unit 110 when centrifugal force is not applied, and may be provided with a valve to regulate the flow of the sample.

Since centrifugal force caused by rotation of the platform 100 is used to transfer the sample from the sample chamber 101 to the sample separation unit 110, the sample separation unit 110 is located radially outward within the microfluidic device, as compared to the position of the sample chamber 101.

The sample separation unit 110 centrifugally separates the sample supplied from the sample chamber 101 into a supernatant and a deposit. For example, if the sample is blood, the supernatant includes blood serum or plasma, and the deposit includes blood corpuscles.

The sample separation unit 110 includes a channel-shaped supernatant collection part 111 extending radially outward from the sample chamber 101, and a deposit collection part 112 located at the end of the supernatant collection part 111 and providing a space to collect the deposit having a large specific gravity. A sample distribution channel 115 is provided on the supernatant collection part 111 to transfer the supernatant obtained by centrifugation to the reaction chamber 160, and a valve 114 is provided within the sample distribution channel 115 to control the flow of the sample. In this exemplary embodiment, the valve 114 may be a closed-type valve which closes a channel so as to prevent the flow of a fluid by driving force transmitted from the outside. Although the exemplary embodiment shown in Figure 2 illustrates the shape of the sample separation unit 110, the sample separation unit 110 may be formed in any of various other shapes. In addition, the sample may be transferred directly to the reaction chamber 160 from the sample chamber 101 in various exemplary embodiments.

A supernatant measurement chamber 116 may be provided between the sample separation unit 110 and the reaction chamber 160 to measure the amount of the supernatant. The supernatant measurement chamber 116 has a volume to accommodate the amount of the supernatant necessary for testing purposes, and a valve 117 may be provided at the exit of the supernatant measurement chamber 116 to control the flow of the fluid. The supernatant measurement chamber 116 is connected to the reaction chamber 160 through a channel. Although not shown in the drawings, a chamber and a channel to accommodate the surplus amount of the liquid sample remaining after testing/measurement may be formed between the sample distribution channel 115 and the supernatant measurement chamber 116. Reaction solutions necessary for reaction in the reaction chamber 160 are accommodated in a first buffer chamber 120 and a second buffer chamber 130. Although the exemplary embodiment shown in FIG. 2 illustrates two buffer chambers, the number of the buffer chambers and kinds of the buffer chambers are not limited thereto, and may be varied according to types of tests to be executed using the microfluidic device.

The following will describe an exemplary blood test using an antigen-antibody reaction.

The first buffer chamber 120 accommodates a first buffer, which may be a conjugate buffer for a sandwich immunoassay, a buffer including a competitive protein for a competitive immunoassay, or a buffer including various enzymes, such as polymerase and a primer for DNA amplification.

A valve 112 may be provided at an exit of the first buffer chamber 120. The valve 112 may be a closed-type valve thereby sealing the first buffer within the first buffer chamber 120 until the valve 112 is opened.

The second buffer chamber 130 may accommodate a substrate buffer that produces a designated color upon reaction with a product of a conjugate reaction or a competitive reaction, or a buffer including various enzymes necessary for a DNA hybridization process. A valve 131 is provided at an exit of the second buffer chamber 130, and when the valve 131 is opened, the second buffer is transferred to the reaction chamber 160.

Although not shown in FIG. 2, the microfluidic device in accordance with this exemplary embodiment may include one or more measurement chambers, each corresponding to the respective buffer chambers to supply predetermined amounts of the respective buffers to the reaction chamber 160. Further, the microfluidic device in accordance with this exemplary embodiment may include excess buffer chambers connected to the measurement chambers to accommodate any amount of the buffers exceeding the predetermined amounts required for testing.

A washing buffer chamber 150 may accommodate a washing buffer to wash the residue after the antigen-antibody reaction. The washing buffer chamber 150 is connected to the reaction chamber 160, and a valve 153 may be provided between the washing buffer chamber 150 and the reaction chamber 160 to control the flow of the washing buffer.

The reaction chamber 160 receives the sample, particularly, the supernatant of the sample, from the supernatant measurement chamber 116 through a channel. Since this exemplary embodiment uses blood as the sample, the supernatant of the sample may be used in the reaction. However, if a sample not requiring centrifugation is tested, the microfluidic device may omit the sample separation unit 110, and the sample accommodated in the sample chamber 101 may be transferred directly to the reaction chamber 160 to participate in the reaction.

A capture material is provided in the reaction chamber 160 for capturing an analyte of the sample. The capture material may specifically react with the analyte in the reaction chamber 160. A marking material is conjugated to the capture material, thereby forming a marking conjugate. The marking conjugate may be selected according to the analyte of interest. If the analyte is an antigen or an antibody, the marking conjugate may be a conjugate in which an antibody or an antigen specifically reacting with the analyte is conjugated with the marking material.

When the reaction in the reaction chamber 160 has been completed, a complex of the analyte and the marking conjugate is generated. For example, the analyte and the marking material may be combined through the capture material. Hereinafter, such a complex will be referred to as a reaction product.

For example, the marking material may be, but is not limited to, latex beads, a metal colloid, such as a gold colloid or a silver colloid, an enzyme, such as peroxidase, a fluorescent material, a noctilucent material or a radioactive isotope. Thus, the kind of the marking material is not limited as long as the marking material discharges a detectable signal representing the presence of the analyte. Hereinafter, for exemplary purposes, a fluorescent material will be used as the marking material. The waste chamber 180 accommodates impurities or reaction residues discharged from the reaction chamber 160. The impurities or reaction residues are washed by the washing buffer of the washing buffer chamber 150 and transferred to the waste chamber 180. The impurities or reaction residues may include any marking conjugate or analyte which has not reacted and therefore has not combined with each other. Such impurities or reaction residues are transferred to the waste chamber 180 so as to improve accuracy in detection in the detection chamber 170. A waste channel 181 is connected to the reaction chamber 160, and a valve 162 is provided within the waste channel 181 so that the flow of the fluid supplied to the waste chamber 180 from the reaction chamber 160 may be controlled.

The detection chamber 170 is connected to the reaction chamber 160 through a valve 164, and receives the final fluid which has completed the reaction from the reaction chamber 160. The final fluid includes the reaction product of the reaction chamber 160, i.e., the complex of the analyte and the marking conjugate. The detection chamber 170 is used to measure the concentration of the analyte. Through the detection chamber 170, the signal detection unit 320 (FIG. 1) may measure the concentration of the analyte by detecting a signal discharged from the marking conjugate combined with the analyte.

The signal detection unit 320 (with reference to FIG. 1) may sense optical characteristics of the analyte, such as fluorescent and light-emitting characteristics or light-absorbing characteristics of the analyte. The signal detection unit 320 includes a light emission part that irradiates light of a specific wavelength to the detection chamber 170, and a light reception part that receives light discharged from the detection chamber 170. Although this exemplary embodiment illustrates the light emission part and the light reception part as being installed adjacent to each other on the same side of the platform 100, the light emission part and the light reception part may be installed at the opposite sides of the platform 100.

In more detail, if fluorescent material is used as the marking material, when excitation light is irradiated onto the detection chamber 170 in which the reaction product is accommodated, the fluorescent material discharges emission light, i.e., a fluorescent signal. Thus, the light emission part of the signal detection unit 320 irradiates excitation light of a specific wavelength, and the light reception part of the signal detection unit 320 receives the fluorescent signal discharged from the fluorescent material. Hereinafter, a signal discharged from the detection chamber 170 in which the marking material is accommodated and detected by the signal detection unit 320 will be referred to as a detection signal, and a signal discharged from the marking material will be referred to as a marking signal. The detection signal includes the marking signal. For example, if a fluorescent material is used as the marking material, the marking signal is referred to as a fluorescent marking signal, and if a radioactive material is used as the marking material, the marking signal is referred to as a radioactive marking signal. As used herein, the terms "fluorescent signal" and "radioactive signal" name generally all fluorescent signals and radioactive signals regardless of materials discharging these signals. A "fluorescent marking signal" and a "radioactive marking signal" are distinguished from the fluorescent signal and the radioactive signal, and represent signals discharged from the fluorescent material and the radioactive material used as the marking material. The detection signal may include a signal discharged from the detection chamber 170 itself, in addition to the marking signal discharged from the marking material, and may also include any signal discharged from foreign substances other than the marking material as long as the signal is detectable by the signal detection unit 320 as the marking signal.

The signal detection unit 320 may detect a signal discharged from the reaction chamber 160. In more detail, a microfluidic device in accordance with another exemplary embodiment may include a plurality of reaction chambers 160, and signals discharged from each of the reaction chambers 160 may be detected when some reaction chambers 160 accommodate reaction products and the remaining reaction chambers 160 do not accommodate reaction products.

A single signal detection unit 320 may be provided so as to detect signals discharged from the respective reaction chambers 160 by rotating the platform 100, or plurality of signal detection units 320 may be provided so as to simultaneously detect signals discharged from the corresponding reaction chambers 160.

Although the embodiment shown in FIG. 2 illustrates that the valves are provided in the channels or at the exits of the various chambers to control the flow of the fluids, other exemplary embodiments may employ any of various types of microfluidic valves. For example, a valve which is opened when pressure of a designated intensity is applied, such as a capillary valve, may be employed, or a valve which is actively operated by power or energy (for example, magnetic energy or heat energy) from the outside by an operating signal may be employed.

As one example of the latter valve, a valve material may be in a solid state at room temperature, and when present on a channel or at an exit of a chamber, closes the channel. The valve material is thereafter melted at a high temperature, and moves to a space within the channel, thereby opening the channel. Energy applied from the outside may be, for example, electromagnetic waves, and an energy source may be a laser light source irradiating a laser beam, a light emitting diode irradiating visible light or ultraviolet light, or a xenon lamp. In case of a laser light source, the energy source may include at least one laser diode.

The external energy source may be selected according to the wavelength of electromagnetic waves which heating particles of the valve material may absorb. Exemplary materials for use as a valve material include, but are not limited to, a thermoplastic resin, such as cyclic olefin copolymer (COC), polymethylmethacrylate (PMMA), polycarbonate (PC), polystyrene (PS), polyoxymethylene (POM), perfluoroalkoxy (PFA), polyvinylchloride (PVC), polypropylene (PP), polyethylene terephthalate (PET), polyetheretherketone (PEEK), polyamide (PA), polysulfone (PSU), or polyvinylidene fluoride (PVDF).

Further, as the valve material, a phase transition material which is in a solid state at room temperature may be employed. The phase transition material may be wax that when heated, is melted and changed to a liquid state. Exemplary waxes include, but are not limited to, paraffin wax, microcrystalline wax, synthetic wax, or natural wax. The phase transition material may be a gel or a thermoplastic resin. As the gel, polyacrylamide, polyacrylate, polymethacrylate or polyvinylamide may be employed. Uniformly dispersed within the valve material may be a plurality of fine heating particles, which absorb electromagnetic wave energy and then generate heat. The fine heating particles have a diameter of about 1 nm to about 100 $\mu$m, so as to effectively pass through a fine channel having a depth of about 0.1 mm and a width of about 1 mm. The fine heating particles have characteristics in that upon absorption of electromagnetic wave energy, for example, by a laser beam, the temperature of the fine heating particles rises rapidly and thus the fine heating particles generate heat. In order to exhibit these characteristics, each of the fine heating particles may include a core including a metal component and a hydrophobic surface structure.

For example, each of the fine heating particles may have a molecular structure including a core formed of Fe, and a plurality of surfactants bonded to and surrounding Fe. The fine heating particles may be dispersed in carrier oil. The carrier oil may be hydrophobic so that the fine heating particles may be uniformly dispersed in the carrier oil. The carrier oil in which the fine heating particles are dispersed is mixed with the molten phase transition material, and then the mixture is injected into the channel or the exit of the chamber and is solidified, thereby closing the channel or the exit of the chamber. The fine heating particles are not limited to the above-described polymer particles, but may be formed in the shape of quantum dots or magnetic beads.

Further, the fine heating particles may be, for example, fine metal oxides, such as $Al_2O_3$, $TiO_2$, $Ta_2O_3$,

Fe2O3, Fe3O4 or HfO2. It is not essential that the valve in accordance with this exemplary embodiment include the fine heating particles. That is, the valve may include only the phase transition material without the fine heating particles, and in such case, a non-contact type heater separated from the microfluidic device by a designated width to melt the valve material may be provided so as to heat the corresponding valve requiring opening.

However, the microfluidic device in accordance with this exemplary embodiment may omit the valve provided at the exit of the chamber or on the channel, and employ various other methods of controlling the flow of fluid without a valve.

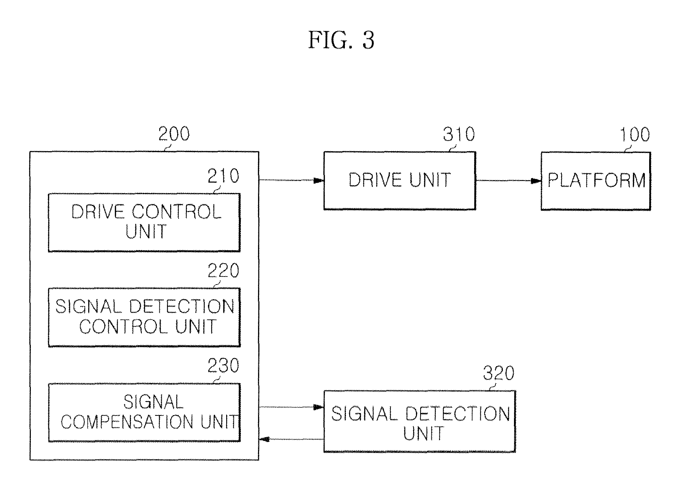

FIG. 3 is a control block diagram of the microfluidic device in accordance with the exemplary embodiment. Hereinafter, use of a fluorescent material as the marking material will be exemplarily described.

With reference to FIG. 3, the microfluidic device in accordance with this exemplary embodiment includes the platform 100 within which the microfluidic structures, such as various chambers and channels, are provided to execute the assay, the drive unit 310 driving the platform 100, the signal detection unit 320 for detecting the concentration of the analyte, and the controller 200 controlling the overall operation of the microfluidic device.

The operation of the platform 100 has been described above in detail with reference to FIGS. 1 and 2, and thus a detailed description thereof will be omitted.

The drive unit 310 provides a driving force to the platform 100 to move the fluid accommodated within the platform 100. If the microfluidic device is in a disc type platform, the drive unit 310 moves the fluid by rotating the platform 100. That is, the drive unit 310 may provide centrifugal force for centrifugation and/or movement of a sample or fluid by rotating the disc-type microfluidic device, and stop or rotate the disc-type microfluidic device so that the detection chamber 170 reaches a designated position. The drive unit 310 may include a motor drive device controlling the angular position of the platform 100. For example, the motor drive device may incorporate a stepped motor or a DC motor.

The signal detection unit 320 detects a signal discharged from the detection chamber 170, as described above. If a fluorescent material is used as the marking material, the signal detection unit 320 irradiates excitation light of a specific wavelength to the detection chamber 170, and receives emission light discharged from the detection chamber 170.

The concentration of the fluorescent material is proportional to the intensity of the emission light. Since the intensity of the emission light is linearly proportional to the intensity of the excitation light, the concentration of the fluorescent material is proportional to the intensity of the emission light (Iemission) normalized by the intensity of the excitation light (Iexcitation), and the concentration (C) of the fluorescent material may be simply expressed by Equation 1 below.

[Equation 1]

$$C = k * (Iemission / Iexcitation)$$

Here, k is a proportional constant, and may have a different value according to characteristics of the microfluidic device.

The intensity of the emission light (Iemission) is influenced only by the fluorescent marking signal discharged from the fluorescent material combined with the analyte, but the emission light detected by the signal detection unit 320 may also include various kinds of background signals. The background signal includes signals other than the fluorescent marking signal among signals included in the detection signal. As described above, the detection signal includes the signal discharged from the detection chamber 170 itself in addition to the fluorescent marking signal discharged from the marking material. In addition, the detection signal includes signals discharged from foreign substances other than the marking material as long as the signals are detectable by the signal detection unit 320. Therefore, the background signal includes these signals.

Particularly, if a fluorescent material is used as the marking material, an auto-fluorescent signal from an interference material is a main factor causing the background signal. Therefore, the controller 200 of the microfluidic device in accordance with this exemplary embodiment executes signal compensation to remove the background signal. Hereinafter, the operation of the controller 200 will be described in detail.

The controller 200 controls the overall operation of the microfluidic device including the rotation of the platform 100, signal detection, etc. The controller 200 includes a drive control unit 210, a signal detection control unit 220, and a signal compensation unit 223 for removing the influence of the background signal to compensate for the detection signal.

In the microfluidic device, the main interference material causing auto-fluorescent light is the detection chamber 170 itself. The detection chamber 170 is formed of plastic, such as PC, PMMA, COC, or PS. Plastic contains a considerable amount of a fluorescent material even if there are differences according to the kinds of plastic, and thus the detection chamber 170 formed of plastic emits a considerable amount of auto-fluorescent light.

Therefore, the signal detection control unit 220 controls the signal detection unit 320 such that the signal detection unit 320 measures a background signal from the detection chamber 170 before the reaction product is transferred from the reaction chamber 160 to the detection chamber 170. At this time, the detection chamber 170 may be vacant, or may accommodate a separate capture material for capturing the reaction product.

Since the background signal includes signals other than the fluorescent marking signal, signals measured before the reaction product is transferred to the detection chamber 170 become the background signals regardless of whether or not the detection chamber 170 accommodates any material. Since the detection chamber 170 itself emits a fluorescent signal, as described above, the background signal may contain a designated amount of the fluorescent signal, and contain a signal generated by a remaining light source component which is not filtered out.

Then the signal detection control unit 220 controls the signal detection unit 320 such that the signal detection unit 320 measures the detection signal from the detection chamber 170 after the reaction product is transferred from the reaction chamber 160 to the detection chamber 170.

The signal compensation unit 230 compensates for the detection signal using the background signal. That is, the signal compensation unit 230 compensates for the detection signal measured after the reaction product is transferred to the detection chamber 170 using the background signal measured before the reaction product is transferred to the detection chamber 170.

Since the detection signal measured after the reaction product is transferred to the detection chamber 170 includes the background signal regardless of the concentration of the analyte, the signal compensation unit 230 removes the background signal from the detection signal and may thus acquire an accurate marking signal, thereby minimizing the influence of the interference material.

A microfluidic device in accordance with another exemplary embodiment may compensate for a detection signal by detecting a signal emitted from a reaction chamber. As described above, the microfluidic device may include a plurality of reaction chambers, and signals discharged from the reaction chambers may be detected under the condition that some reaction chambers accommodate reaction products and the remaining reaction chambers do not accommodate reaction products. Here, a signal detected from the reaction chambers in which the reaction products are not accommodated becomes a background signal, and a signal detected from the reaction chambers in which the reaction products are accommodated becomes a detection signal. The signal detection unit 230 may execute signal compensation by removing the background signal from the detection signal.

Further, a microfluidic device in accordance with another exemplary embodiment may include a plurality of detection chambers, and signals discharged from the detection chambers may be detected under the condition that some detection chambers accommodate reaction products and the remaining detection chambers do not accommodate reaction products. Here, a signal detected from the detection chambers in which the reaction products are not accommodated becomes a background signal, and a signal detected from the detection chambers in which the reaction products are accommodated becomes a detection signal. The signal detection unit 230 may execute signal compensation by removing the background signal from the detection signal.

Accordingly, in the microfluidic device in accordance with the exemplary embodiment, chambers, from which signals are detected, may be reaction chambers or detection chambers. That is, the kinds or types of chambers, from which signals are detected, are not limited. Further, a chamber, from which a background signal is detected and a chamber, from which a detection signal is detected, may be the same chamber or may be different chambers, as long as the chamber does not contain the reaction product when the background signal is detected, and does contain the reaction product when the detection signal is detected from the chamber. Hereinafter, various exemplary embodiments to execute more accurate signal compensation through the signal compensation unit 230 will be described.

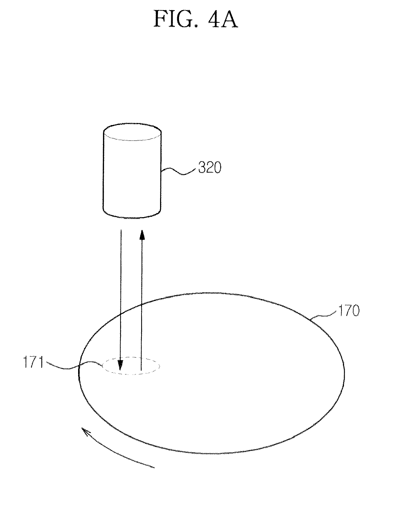

FIGS. 4A to 4C are views illustrating measurement of fluorescent signals at different detection positions of the same detection chamber 170. In order to clearly represent the relations between the signal detection unit 320 and the detection chamber 170, constituent elements of the microfluidic device, except for the signal detection unit 320 and the detection chamber 170, are omitted in FIGS. 4A to 4C.

The fluorescent material serving as the marking material may be distributed in respective regions of the detection chamber 170 at different concentrations. Therefore, if a signal is detected from one region of the detection chamber 170, accuracy may be lowered. Thus, the signal detection control unit 220 controls the signal detection unit 320 such that the signal detection unit 320 measures signals at different detection positions of the detection chamber 170 a plurality of times.

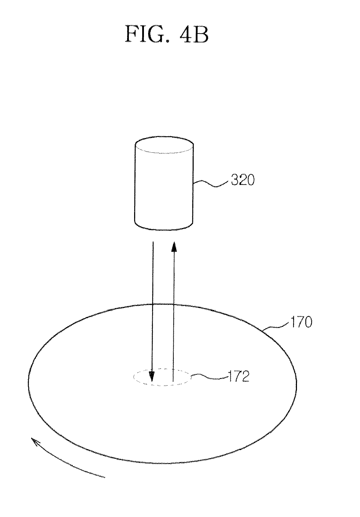

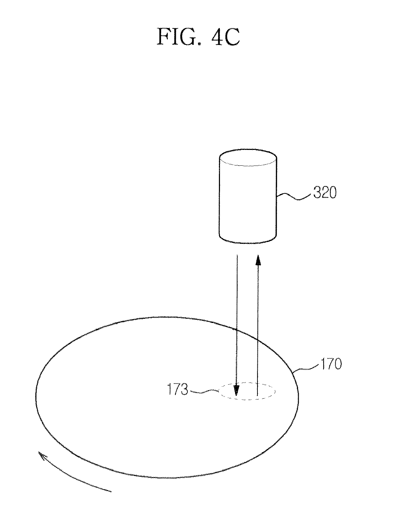

The signal detection unit 320 detects a signal at a first detection position 171 within the detection chamber 170, as shown in FIG. 4A, detects a signal at a second detection position 172 within the detection chamber 170, as shown in FIG. 4B, and detects a signal at a third detection position 173 within the detection chamber 170, as shown in FIG. 4C.

As shown in FIGS. 4A to 4C, the first to third detection positions 171, 172 and 173 are different, and the detection positions 171, 172 and 173 may be changed by rotating or vibrating the platform 100 with the drive unit 310 under the control of the drive control unit 210.

When signal detection has been completed a plurality of times, the signal compensation unit 230 compensates for the detection signal. The detection signal to be compensated for is the mean value of the signals detected over the plurality of times. Accuracy and reproducibility in analysis may be increased by executing signal detection at different positions within detection chamber 170 a plurality of times and compensating for the mean value of the detection signals, as in this exemplary embodiment.

Although the embodiment shown in FIGS. 4A to 4C executes signal detection three times, the detection number of signals is not limited thereto. The number of detection of signals may be determined in consideration of accuracy in analysis and efficiency of inspection.

Further, since the fluorescent material transferred from the reaction chamber 160 to the detection chamber 170 remains in the liquid state for a certain amount of time within the detection chamber 170, the detected concentration in a particular region within the detection chamber 170 may vary with the passage of time. Therefore, detection of a signal at the same position may be executed at different times, and the mean value of the detection signals may be compensated for.

Further, after a designated amount of time has elapsed, fluidity of the fluorescent material transferred from the reaction chamber 160 to the detection chamber 170 decreases, thereby stabilizing the fluorescent material. Therefore, after a designated time from transfer of the fluorescent material to the detection chamber 170 has elapsed, detection of a signal may be executed. In this context, the term "designated time" means the time required for the fluorescent material to decrease in fluidity and become stabilized. Such designated time may be determined experimentally and statistically.

Further, it is possible to uniformly distribute the concentration of fluorescent material within the detection chamber 170 without execution of signal detection a plurality of times or waiting for stabilization of the concentration distribution. For this purpose, the platform 100 may be vibrated up and down or left and right after the fluorescent material is transferred to the detection chamber 170. Then, signal detection may be executed immediately after, or be executed after the flow of the fluorescent material is considerably stopped, resulting in stabilization of the fluorescent material.

The above-described exemplary embodiments are to increase accuracy and reproducibility in analysis, and it should be understood that two or more of these exemplary embodiments may be combined.

As another factor lowering accuracy in a conventional analysis result, there may be crosstalk between the detection chambers 170. Such crosstalk is generated from inclusion of a plurality of detection chambers 170 within the microfluidic device. For example, light irradiated to a specific detection chamber 170 may be partially irradiated to other detection chambers 170 due to refraction, reflection and/or scattering, and fluorescent light generated at this time may enter the light reception part of the signal detection unit 320. Such crosstalk may induce errors in concentration analysis, particularly when a low concentration sample and a high concentration sample are present.

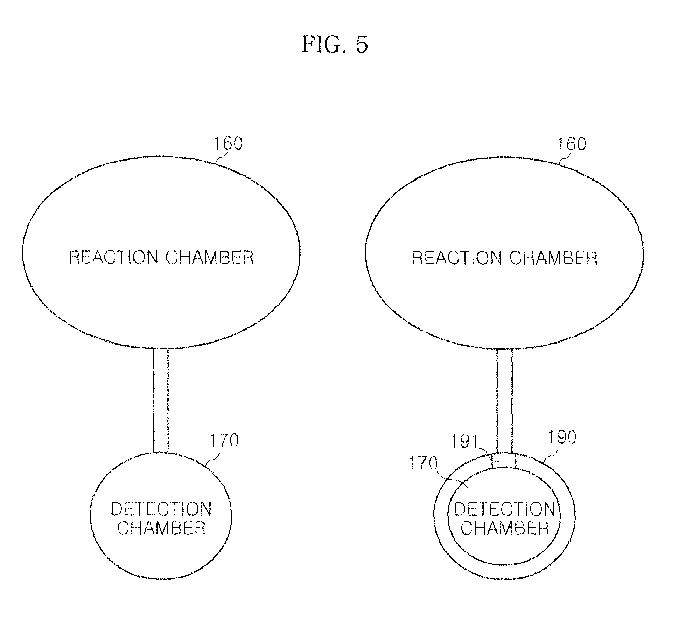

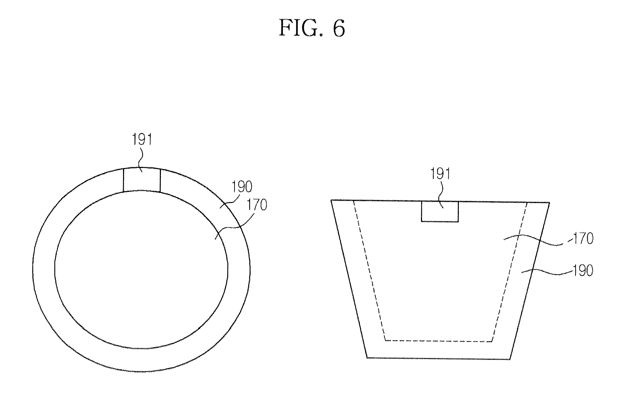

Therefore, a microfluidic device in accordance with an exemplary embodiment may include a cartridge in a shape sufficient for surrounding each of the detection chambers 170. The cartridge may be formed of an opaque material to effectively suppress crosstalk of fluorescent signals between the detection chambers 170. FIGS. 5 and 6 are views illustrating the detection chamber 170 surrounded by a cartridge in accordance with an exemplary embodiment. Hereinafter, this exemplary embodiment will be described in detail with reference to FIGS. 5 and 6.

In FIG. 5, a detection chamber 170 not provided with a cartridge and the reaction chamber 160 are illustrated at left, while a detection chamber 170 provided with a cartridge 190 and the reaction chamber 160 are illustrated at right. FIG. 5 is a plan view.

The cartridge 190 surrounding the detection chamber 170 may be formed in a color which is opaque and/or absorbs a considerable amount of light. For example, if the cartridge 190 is formed in black, the cartridge 190 absorbs most of the light irradiated to the detection chamber 170 and may thus prevent light from reaching other detection chambers 170.

Further, the cartridge 190 may be formed in a color which reflects a considerable amount of light. For example, if the cartridge 190 is formed in white or silver, the cartridge 190 reflects excitation light irradiated to the detection chamber 170 and may thus prevent light from reaching other detection chambers 170.

In FIG. 6, the plan view of the detection chamber 170 surrounded by the cartridge 190 is illustrated at left, and a longitudinal-sectional view of the detection chamber 170 surrounded with the cartridge 190 is illustrated at right.

The cartridge 190 provided in this exemplary embodiment is provided with a channel 191 through which the final fluid including the reaction product flows into the detection chamber 170 from the reaction chamber 160, as shown in FIG. 6. Although FIG. 6 illustrates only the inflow channel 191, the cartridge 190 may be further provided with a discharge channel if there is another chamber into which the fluid of the detection chamber 170 is transferred. Thus, the number of inflow channels or discharge channels corresponds to the number other chambers connected to the detection chamber 170.

The cartridge 190 may be formed of plastic, such as PC, COC, PMMA, or PS. However, the cartridge 190 is not limited to the above materials, and may be formed of any material having properties such as chemical and biological stability, optical transparency, and mechanical processability.

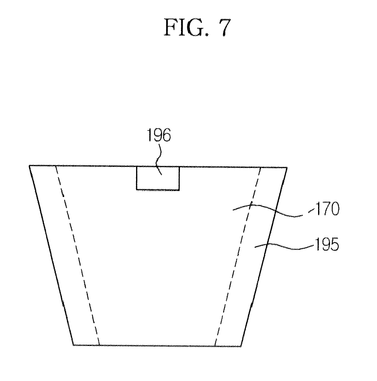

Meanwhile, the cartridge may be implemented in a form having a bottom thereof perforated. FIG. 7 is a cross-sectional view illustrating the cartridge in accordance with another exemplary embodiment. Although the light emission part and the light reception part of the signal detection unit 320 shown on FIGS. 4A to 4C are illustrated as being installed on the same side of the platform 100, the light emission part and the light reception part may be installed while facing each other. That is, one of the light emission part and the light reception part may be installed at an upper side of the platform 100 while the remaining of the light emission part and the light reception part may be installed at a lower side of the platform 100. For example, if the light emission part is installed at the upper side of the platform 100, and the light reception part is installed at the lower side of the platform 100, the light irradiated from the light emission part reaches the light reception part after passing through the detection chamber 170. With reference to Fig. 7, to this end, a cartridge 195 is implemented in the form having a bottom thereof perforated such that light passes through the detection chamber 170 upward and downward. As for the circumference of the detection chamber 17, light is blocked or reflected by the cartridge 195, so the light being irradiated from the signal detection unit 320 is prevented from reaching another chamber. The cartridge 195 provided in this exemplary embodiment is provided with a channel 196 through which the final fluid including the reaction product flows into the detection chamber 170 from the reaction chamber 160, as shown in FIG. 7. Although FIG. 7 illustrates only the inflow channel 196, the cartridge 195 may be further provided with a discharge channel if there is another chamber into which the fluid of the detection chamber 170 is transferred. Thus, the number of inflow channels or discharge channels corresponds to the number other chambers connected to the detection chamber 170.

Although the above exemplary embodiment describes the microfluidic device as further including the cartridge 190 surrounding the detection chambers 170, the detection chambers 170 themselves may be formed in a color which does not transmit light without the use of a separate cartridge. For example, if the detection chambers 170 are formed in black, white or silver, the detection chambers 170 may prevent light irradiated to the detection chambers 170 from reaching other detection chambers 170.

Further, although the above exemplary embodiment describes the cartridge 190 as surrounding the detection chambers 170, a cartridge may surround the reaction chambers 160 and/or the waste chambers 180, or surround some or all of these chambers. Since a fluorescent material may be present in the reaction chambers 160 or in the waste chambers 180 and such a fluorescent material may serve as an interference material to the signal detection unit 320, generation of crosstalk may be prevented if the reaction chambers 160 or the waste chambers 180 are surrounded with the cartridge.

Of course, the reaction chambers 160 or the waste chambers 180 themselves may be formed in a color which does not transmit light.

Hereinafter, control methods of a microfluidic device in accordance with exemplary embodiments will be described. In these exemplary embodiments, only signal detection after combination of the analyte and the marking conjugate within the reaction chamber 160 has been completed, will be described.

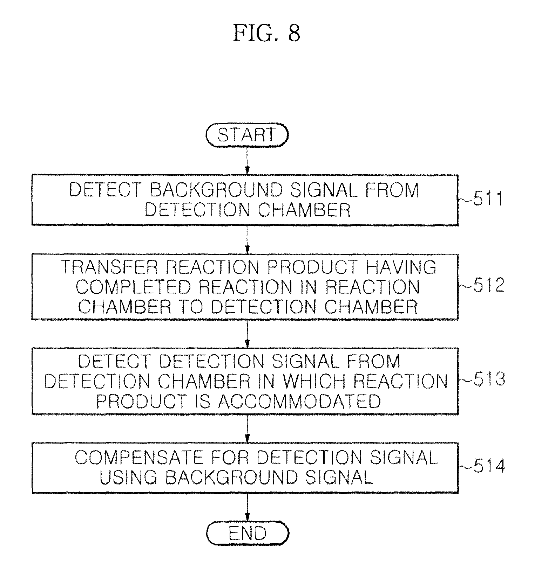

FIG. 8 is a flowchart illustrating a control method of a microfluidic device in accordance with an exemplary embodiment.

With reference to FIG. 8, a background signal is detected from the detection chamber 170 (Operation 511) prior to transfer of a reaction product therein. Thus, the detection chamber 170 is in a state in which a reaction product is not yet accommodated in the detection chamber 170, and the background signal may include a fluorescent signal emitted from the detection chamber 170 itself and/or any remaining light source component which is not filtered out.

When the detection of the background signal has been completed, the reaction product having completed a reaction within the reaction chamber 160 is transferred to the detection chamber (Operation 512). The reaction product is a complex of the analyte and the marking conjugate, and the marking conjugate is a complex of the capture material of the analyte and the marking material.

Thereafter, a detection signal is detected from the detection chamber 170 within which the reaction product is accommodated (Operation 513). If a fluorescent material is used as the marking material of the marking conjugate, then when the light emission part of the signal detection unit 320 irradiates excitation light of a specific wavelength to the detection chamber 170, the fluorescent material emits a fluorescent marking signal after absorbing energy of the excitation light. Thereafter, the light reception part of the signal detection unit 320 detects the fluorescent marking signal.

Thereafter, the detection signal is compensated for using the detected background signal (Operation 514). Since the background signal includes the fluorescent signal emitted from the detection chamber 170 itself and/or a signal generated due to any light source component which is not filtered out, the detection signal may be compensated for by removing the background signal from the detection signal. Accordingly, an accurate fluorescent marking signal is acquired.

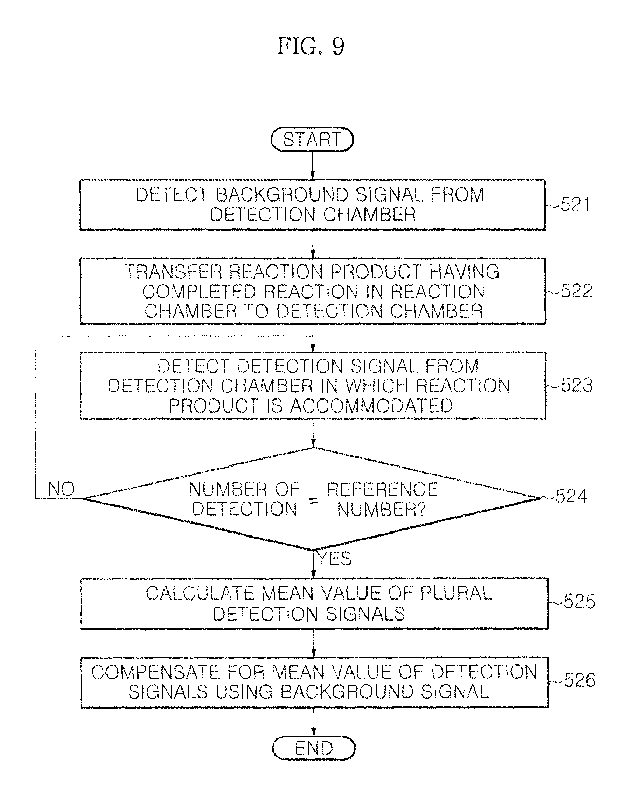

FIG. 9 is a flowchart illustrating a control method of a microfluidic device in accordance with another exemplary embodiment.

With reference to FIG. 9, a background signal is detected from the detection chamber 170 (Operation 521), and a reaction product having completed a reaction within the reaction chamber 160 is transferred to the detection chamber 170 (Operation 522).

Thereafter, signal detection from the detection chamber 170 in which the reaction product is accommodated is executed (Operation 523). In order to improve accuracy in analysis, signal detection is executed a plurality of times. After a detection signal has been detected, the controller 200 determines whether or not the number of signal detection reaches a predetermined reference number (Operation 524). Upon determining that the number of signal detection does not equal the predetermined reference number ("No" in Operation 524), signal detection from the detection chamber 170 is re-executed (Operation 523). Here, the reference number may be determined by a user in consideration of various aspects, e.g., accuracy in analysis and efficiency of inspection.

When signal detection is execute a plurality of times, signals may be detected at the same detection position within the detection chamber 170, or may be detected at different detection positions within the detection chamber 170 by rotating and/or vibrating the platform 100.

Upon determining that the number of signal detections has reached the predetermined reference number ("Yes" in Operation 524), the mean value of the plurality of detection signals is calculated (Operation 525). Thereafter, the mean value of the detection signals is compensated for using the above-detected background signal (Operation 526). For example, the mean value of the detection signals may be compensated for by removing the background signal from the mean value of the detection signals.

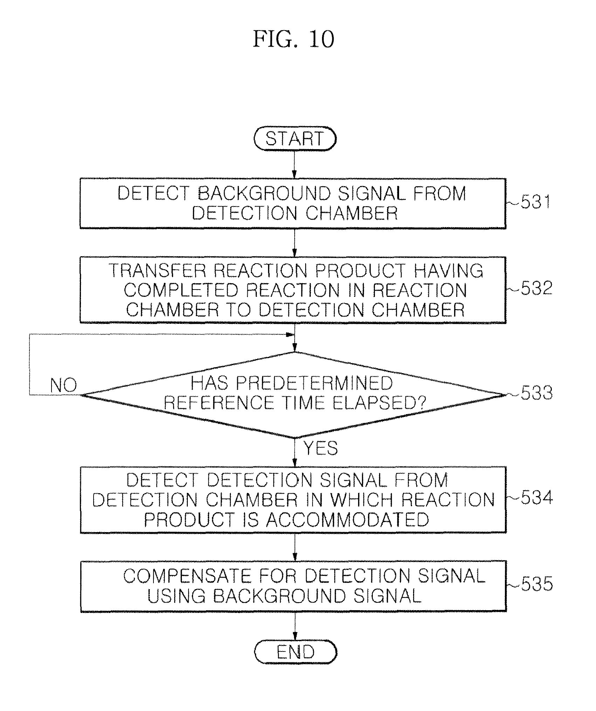

FIG. 10 is a flowchart illustrating a control method of a microfluidic device in accordance with another exemplary embodiment.

With reference to FIG. 10, a background signal is detected from the detection chamber 170 (Operation 531), and a reaction product having completed a reaction within the reaction chamber 160 is transferred to the detection chamber 170 (Operation 532).

Thereafter, the controller 200 determines whether or not a predetermined reference time has elapsed (Operation 533). Here, the reference time means a time required for the reaction product transferred to the detection chamber 170 to decrease in fluidity and thus become stabilized. Such time required for decreasing fluidity may be predetermined experimentally and statistically.

Upon determining that the predetermined reference time has elapsed ("Yes" in Operation 533), a detection signal is detected from the detection chamber 170 within which the reaction product is accommodated (Operation 534). Thereafter, the detection signal is compensated for using the above-detected background signal (Operation 535). The compensation of the signal may be executed by removing the background signal from the detection signal, as described above.

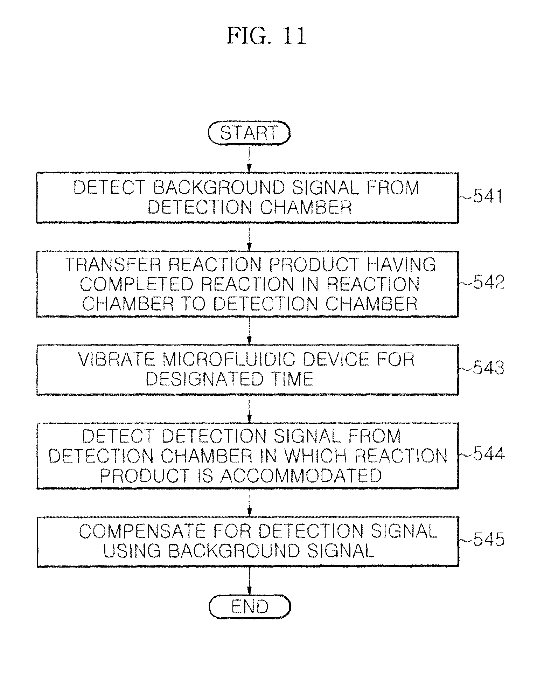

FIG. 11 is a flowchart illustrating a control method of a microfluidic device in accordance with yet another exemplary embodiment.

With reference to FIG. 11, a background signal is detected from the detection chamber 170 (Operation 541), and a reaction product having completed a reaction within the reaction chamber 160 is transferred to the detection chamber 170 (Operation 542).

Thereafter, the microfluidic device is vibrated for a designated amount of time (Operation 543), which serves to uniformly distribute the reaction product within the detection chamber 170. The designated time required for uniform distribution of the transferred reaction product may be predetermined by a user in consideration of accuracy in analysis and efficiency of inspection. Vibration of the microfluidic device means that the drive control unit 210 transmits a control signal to the drive unit 310 so that the drive unit vibrates the platform 100.

Thereafter, a detection signal is detected from the detection chamber 170 within which the reaction product is accommodated (Operation 544), and the detection signal is compensated for by using the above-detected background signal (Operation 545). Compensation of the signal may be accomplished by removing the background signal from the detection signal, as described above.

Although the exemplary embodiments shown in FIGS. 8 to 11 illustrate that the background signal is detected from the detection chamber in which the reaction product is not accommodated, and the detection signal is detected from the detection chamber within which the reaction product is accommodated, exemplary embodiments described herein are not limited thereto. For example, the microfluidic device may include a plurality of reaction chambers, and a detection signal may be detected from reaction chambers in which reaction products are accommodated, while the background signal may be detected from any one or more of the remaining reaction chambers in which reaction products are not accommodated. Further, the microfluidic device may include a plurality of detection chambers, and a detection signal may be detected from detection chambers in which reaction products are accommodated, while the background signal may be detected from any one or more of the remaining detection chambers in which reaction products are not accommodated.

As is apparent from the above description, in a microfluidic device and a control method in accordance with an exemplary embodiment, a background signal is measured before a reaction product is accommodated in a chamber and a signal measured after the reaction product is accommodated in the chamber is compensated for using the background signal, thereby improving reliability of the test results.

Further, an opaque cartridge surrounding the chamber may be provided, thereby preventing a signal generated from the reaction product accommodated in the chamber from influencing signal detection from other chambers.

Although a few exemplary embodiments have been shown and described, it would be appreciated by those skilled in the art that changes may be made in these embodiments without departing from the principles and spirit of the invention, the scope of which is defined in the claims and their equivalents.

**Claims**

1. A control method of a microfluidic device comprising:

   detecting a background signal from a first chamber of the microfluidic device, while the first chamber does not contain a reaction product that is a complex of an analyte and a marking material;

detecting a detection signal from a second chamber of the microfluidic device, while the second chamber contains the reaction product; and

compensating for the detection signal using the background signal from the detection signal.

2. The control method according to claim 1, wherein the first chamber and the second chamber are the same chamber.

3. The control method according to claim 1, wherein the first chamber and the second chamber are different chambers.

4. The control method according to claim 2, further comprising transferring the reaction product to the second chamber, after the detection of the background signal from the first chamber in which the reaction product is not accommodated.

5. The control method according to claim 1, wherein the first and second chambers are reaction chambers or detection chambers.

6. The control method according to claim 1, wherein the marking material is one selected from the group consisting of a fluorescent material, a metal colloid, an enzyme, latex beads, a noctilucent material and a radioactive isotope.

7. The control method according to claim 1, wherein the compensation of the detection signal includes removing the background signal from the detection signal.

8. The control method according to claim 4, further comprising vibrating the microfluidic device to uniformly distribute the marking material transferred to the second chamber, before the detecting of the detection signal.

9. The control method according to claim 4, wherein the detection signal is detected after a designated time from transfer of the reaction product to the second chamber has elapsed.

10. The control method according to claim 9, wherein the designated time corresponds to a time taken to stabilize the flow of the marking material transferred to the second chamber.

11. The control method according to claim 1, further comprising:

detecting the detection signal from the second chamber i a plurality of times; and

calculating the mean value of the detected detection signals,

wherein the detection signal to be compensated for is the calculated mean value of the detection signals.

12. A microfluidic device comprising:

a platform including a first chamber which does not contain a reaction product, and a second chamber which contains the reaction product, wherein the reaction product is a complex of an analyte and a marking material;
a signal detection unit that is configured to detect signals emitted from the first chamber and the second chamber; and
a controller that is configured to compensate for the signal detected from the second chamber using the signal detected from the first chamber by the signal detection unit.

13. The microfluidic device according to claim 12, wherein the first chamber and the second chamber are reaction chambers.

14. The microfluidic device according to claim 12, wherein the controller removes the signal detected from the first chamber from the signal detected from the second chamber.

15. The microfluidic device according to claim 12, wherein the controller controls the signal detection unit so as to detect the signal from at least two different detection positions of the second chamber in which the reaction product is accommodated plural times, and compensates for a mean value of the plural detection signals.

FIG. 1

FIG. 2

FIG. 3

FIG. 4A

FIG. 4B

FIG. 4C

FIG. 5

160

REACTION CHAMBER

170

DETECTION
CHAMBER

160

REACTION CHAMBER

191    190

170

DETECTION
CHAMBER

FIG. 6

FIG. 7

FIG. 8

```
           ┌─────────┐
           │  START  │
           └────┬────┘
                │
  ┌─────────────▼──────────────┐
  │  DETECT BACKGROUND SIGNAL FROM  │ ~511
  │     DETECTION CHAMBER           │
  └─────────────┬──────────────┘
                │
  ┌─────────────▼──────────────┐
  │  TRANSFER REACTION PRODUCT HAVING │ ~512
  │  COMPLETED REACTION IN REACTION   │
  │  CHAMBER TO DETECTION CHAMBER     │
  └─────────────┬──────────────┘
                │
  ┌─────────────▼──────────────┐
  │  DETECT DETECTION SIGNAL FROM        │ ~513
  │  DETECTION CHAMBER IN WHICH REACTION │
  │  PRODUCT IS ACCOMMODATED             │
  └─────────────┬──────────────┘
                │
  ┌─────────────▼──────────────┐
  │  COMPENSATE FOR DETECTION SIGNAL │ ~514
  │  USING BACKGROUND SIGNAL         │
  └─────────────┬──────────────┘
                │
           ┌────▼────┐
           │   END   │
           └─────────┘
```

FIG. 9

START

DETECT BACKGROUND SIGNAL FROM
DETECTION CHAMBER ~521

TRANSFER REACTION PRODUCT HAVING
COMPLETED REACTION IN REACTION
CHAMBER TO DETECTION CHAMBER ~522

DETECT DETECTION SIGNAL FROM
DETECTION CHAMBER IN WHICH REACTION
PRODUCT IS ACCOMMODATED ~523

NO    NUMBER OF   =   REFERENCE
      DETECTION       NUMBER? ~524

YES

CALCULATE MEAN VALUE OF PLURAL
DETECTION SIGNALS ~525

COMPENSATE FOR MEAN VALUE OF DETECTION
SIGNALS USING BACKGROUND SIGNAL ~526

END

FIG. 10

```
                        ( START )
                            │
                            ▼
            ┌─────────────────────────────────┐
            │  DETECT BACKGROUND SIGNAL FROM   │
            │       DETECTION CHAMBER          │ ~531
            └─────────────────────────────────┘
                            │
                            ▼
            ┌─────────────────────────────────┐
            │ TRANSFER REACTION PRODUCT HAVING │
            │  COMPLETED REACTION IN REACTION  │ ~532
            │  CHAMBER TO DETECTION CHAMBER    │
            └─────────────────────────────────┘
                            │
          ┌─────────────────┤
          │                 ▼
          │          ◇─────────────────◇
      NO  │         ╱  HAS PREDETERMINED  ╲
          └────────◇   REFERENCE TIME ELAPSED? ◇ ~533
                    ╲                   ╱
                     ◇─────────────────◇
                            │ YES
                            ▼
            ┌─────────────────────────────────┐
            │    DETECT DETECTION SIGNAL FROM  │
            │ DETECTION CHAMBER IN WHICH REACTION │ ~534
            │    PRODUCT IS ACCOMMODATED       │
            └─────────────────────────────────┘
                            │
                            ▼
            ┌─────────────────────────────────┐
            │ COMPENSATE FOR DETECTION SIGNAL  │
            │    USING BACKGROUND SIGNAL       │ ~535
            └─────────────────────────────────┘
                            │
                            ▼
                        ( END )
```

FIG. 11

START

DETECT BACKGROUND SIGNAL FROM
DETECTION CHAMBER ~541

TRANSFER REACTION PRODUCT HAVING
COMPLETED REACTION IN REACTION
CHAMBER TO DETECTION CHAMBER ~542

VIBRATE MICROFLUIDIC DEVICE FOR
DESIGNATED TIME ~543

DETECT DETECTION SIGNAL FROM
DETECTION CHAMBER IN WHICH REACTION
PRODUCT IS ACCOMMODATED ~544

COMPENSATE FOR DETECTION SIGNAL
USING BACKGROUND SIGNAL ~545

END